# EUROPEAN PATENT APPLICATION

(11) **EP 1 674 088 A1**
(43) Date of publication of application: **28.06.2006**
(21) Application number: 04792796.7
(22) Date of filing: 15.10.2004
(51) Int. Cl.: A61K 31/155, A61K 47/04, A61K 47/20, A61K 47/36, A61K 9/06, A61P 3/10

(54) **JELLY PREPARATION CONTAINING BIGUANIDE MEDICINE**

(30) Priority: 17.10.2003 JP 2003358136
(71) Applicant: Medrx Co., Ltd., Higashikagawa-shi, Kagawa 769-2702 (JP); Nippon Shinyaku Co., Ltd., Kyoto-shi, Kyoto 601-8550 (JP)
(72) Inventor: YOKOYAMA, Hideakira, Tokushima-shi, Tokushima 770-0866 (JP); HIRATA, Akihiko, Naruto-shi, Tokushima 772-0017 (JP); HAMAMOTO, Hidetoshi, Itano-gun, Tokushima 771-0207 (JP); ISHIBASHI, Masaki, Naruto-shi, Tokushima 772-0001 (JP); YAMASAKI, Keiko, Higashikagawa-shi, Kagawa 769-2515 (JP); FUJII, Takeru, Naruto-shi, Tokushima 772-0051 (JP)
(74) Representative: Müller-Boré & Partner Patentanwälte
(86) International application number: PCT/JP2004/015654
(87) International publication number: WO 2005/037264

(57) **Abstract**

The invention provides a biguanide drug-containing jelly preparation of which discomfort upon administration is decreased by the control of its harshness or bitterness. In addition, the preparation has stability and excellent ability for releasing a drug in the digestive tract. The biguanide drug-containing jelly preparation of the invention is **characterized by** comprising a biguanide drug, an inorganic acid, and a water-soluble polymer. The jelly preparation of the invention is excellent in both stability and ability for releasing a drug, which are usually incompatible characters, particularly by the action of the inorganic acid.

## Description

### TECHNICAL FIELD

The present invention relates to a jelly preparation comprising a biguanide drug such as metformin hydrochloride and buformin hydrochloride.

### BACKGROUND ART

Diabetes mellitus is classified into type 1 diabetes mellitus and type 2 diabetes mellitus depending on the cause of the disease, and in recent years, type 2 diabetes mellitus (non-insulin-dependent diabetes mellitus) becomes problematic as an adult disease. For therapy of the disease, lowering blood sugar level is effective, and therapy of type 1 diabetes mellitus is performed mainly by administering insulin. On the other hand, type 2 diabetes mellitus is treated mainly by an oral hypoglycemic agent, since the condition in which the action of insulin is not achieved although insulin is secreted (insulin resistance) is involved significantly in the onset of type 2 diabetes mellitus, and insulin must be administered by subcutaneous injection which gives a pain to the patient.

Although a sulfonylurea or a sulfonamide agent is exemplified as such an oral hypoglycemic agent, a biguanide drug is mainly used worldwide from the viewpoint of cost and effect. For example, metformin, a typical example of a biguanide drug, has sales next to insulin on the market of diabetes drug. However, a biguanide drug has a problem of difficulty in being taken.

That is, a biguanide drug is highly water-soluble and is considered to rapidly dissolve in saliva in the mouth, and the resultant solution has a strong harsh and bitter taste. In addition, its dose is generally high. For example, the amount of metformin hydrochloride to be administered per dose is considerably as high as 250 mg in Japan or 850 mg in the US. Accordingly, a variety of biguanide drug-containing preparations for easily securing compliance by reducing patient's discomfort upon administration have been developed.

For example, the harsh or bitter taste of a biguanide drug can be solved by coating its tablet or granule, or converting it into microcapsules, thereby completely preventing the drug from contacting with taste buds.

However, in application of such solid preparations to a biguanide drug to be administered in a large amount, there remains the problem of difficulty in swallowing. That is, irritation or pain resulting from contact of the solid preparation with the oral cavity or with the larynx and pharynx, or a physical injury caused upon rubbing of the solid preparation against mucous membrane tissues, can give discomfort to a patient. If a patient daily takes a large amount of water at the time of administration to reduce these discomforts, water itself may cause discomfort or eventual aspiration (eventual aspiration to lung).

Such problem is more serious for patients particularly having difficulty in swallowing, such as patients of advanced age. For such patients, a liquid preparation or a syrup preparation which can be easily administered is preferable, but cannot solve the problem of eventual aspiration yet. Moreover, the harsh or bitter taste of a biguanide drug appears when it is solubilized. That is, in the case of metformin hydrochloride as a typical biguanide drug, although free metformin is sparingly soluble and has thus no harsh and bitter taste, it will taste harsh and bitter significantly when made readily soluble by conversion into salts.

As a method of relieving such harsh or bitter taste of a liquid preparation of a biguanide drug for internal use, a method that involves adding an organic acid such as malic acid is disclosed in Japanese translation of PCT international publication No. 2002-512953.

However, even if the harsh and bitter taste is relieved, a liquid preparation cannot solve problems such as eventual aspiration. Further, even if an organic acid is added, its effect is not satisfactory when a biguanide drug is inevitably contacted with taste buds. In this respect, a jelly preparation is considered more effective than a liquid preparation.

A jelly preparation of a biguanide drug is also disclosed in Example 9 of the above publication No. 2002-512953. However, a jelly preparation is generally poor in stability and is liable to cause syneresis (separation of water from gel layer) in acidic region. According to the inventors' experiment, the jelly preparation described in the above patent publication is difficult to be gelled. This is possibly because the gelling of gelatin as a gel base is prevented by malic acid as organic acid. Accordingly, when the gelatin concentration is increased to accelerate gelling while an organic acid is added, the stability of the gel is increased, but the ability for releasing a drug is decreased.

To solve the problems of the prior art described above, use of alginic acid or pectin gelling by acidity as a gel base together with an organic acid can be anticipated. A jelly preparation using these bases achieves certain improvement in stability, but there still remains the problem of a reduction in ability for releasing a drug in the digestive tract. That is, the jelly preparation which is inherently solidified in acidic region will further increase its strength by the action of a strong acid gastric juice in the stomach after administration. As the result, the ability for releasing a drug in the digestive track is further deteriorated.

On one hand, it is thought that the problem described above can be solved by preparing a jelly preparation in the vicinity of neutrality. However, when the pH of the preparation is made higher than 6, the discomfort of the preparation described in the above publication No. 2002-512953 is increased and its stability is deteriorated.

### DISCLOSURE OF INVENTION

Under these circumstances described above, since,the highest priority should be given to the reduction in harsh and bitter taste in order to facilitate administration of a biguanide drug, the jelly preparation of a biguanide prepared in acidic region is preferable. Actually, a biguanide drug-containing jelly preparation using a fruit acid i.e. malic acid as an organic acid is prepared in Example 9 in the above publication No. 2002-512953.

However, the jelly preparation according to this prior art is not easily gelled. It follows that when the concentration of gelatin as a gel base is increased or a base gelling in acidic region is used, the stability can be improved, but the ability for releasing a drug is worsened. When an organic, acid is used as a masking agent, a texture having excellent in feeling upon eating such as softness can be obtained, but a reduction in the ability for releasing a drug results. When a jelly preparation is prepared in acidic region, there is also the problem of deterioration in stability due to easily occurring syneresis. Accordingly, it is desirable that a biguanide drug-containing preparation not only solves the problem of discomfort upon administration recognizedin the above publication No.2002-512953, but also simultaneously satisfies two contradictory characteristics, that is, stability and excellent ability for releasing a drug after administration.

It is an object of the present invention to provide a biguanide drug-containing jelly preparation of which discomfort on administration is decreased by the control of its harshness or bitterness, and which is excellent in stability and also in ability for releasing a drug in the digestive tract.

To solve the problem described above, the present inventors prepared a wide variety of biguanide drug-containing jelly preparations and made extensive study on a preparation of which discomfort upon administration is significantly decreased and having characteristics considered contradictory to each other, that is, stability and excellent ability for releasing a drug in the body. As a result, the present inventors found that when a water-soluble polymer is used in forming a jelly preparation, the discomfort upon administration of the preparation can be decreased, and concomitantly eventual aspiration upon administration can also be eliminated because the jelly preparation has a loose crosslinkage of the water-soluble polymer. In addition, the present inventors found that an addition of inorganic acid, not only suppresses the harsh and bitter taste of a biguanide drug, but also improve significantly the stability and ability for releasing a drug of the jelly preparation. As the result, the present inventions were thereby completed.

A biguanide drug-containing jelly preparation of the present invention, comprises a biguanide drug, an inorganic acid, and a water-soluble polymer.

The ratio by weight of the inorganic acid to the biguanide drug is in the range of 0.01 to 2. This is because when the amount of the inorganic acid added is lower than the range, the bitterness or the like of the biguanide drug may not be sufficiently suppressed in some cases, while when the amount is higher than the range, the stability of the jelly may be worsened in some cases.

The pH of the preparation in a form of a solution just before gelation is preferably 4.0 or more. This is because when the pH is 4.0 or more at the time of adding the inorganic acid to the jelly preparation, syneresis hardly occurs thus improving the stability of the jelly preparation.

The inorganic acid is preferably phosphoric acid, hydrochloric acid, sulfuric acid, lower alkyl sulfonic acid, or a mixture of two or more selected therefrom. This is because these inorganic acids are adopted in consideration of pharmaceutical safety, and their effects, by which the discomfort caused by the biguanide drug can be decreased and the ability for releasing a drug of the preparation is prevented from deteriorating, are demonstrated in the Examples shown later.

The inorganic acid is particularly preferably phosphoric acid. When phosphoric acid is used as the inorganic acid, further addition of phosphate is preferable. When phosphoric acid is used in a sufficient amount as an agent for masking the bitter taste of the biguanide drug, the discomfort upon administration and ability for releasing a drug can be improved. However, when the pH measured just before gelation of the jelly preparation is too low, the stability of the preparation may be deteriorated in some cases. Accordingly, when both phosphoric acid and phosphate are added, the ability for releasing a drug and stability of the preparation and the discomfort upon administration can be simultaneously improved.

As the water-soluble polymer, it is preferable to use alginic acid, pectin, gellan gum, agar, carrageenan, carob bean gum, tamarind gum, pullulan, or a mixture of two or more selected therefrom. This is because these water-soluble polymers are pharmaceutically safe and excellent as a constituent component of the jelly preparation of the invention that is excellent in safety and ability for releasing a drug.

The biguanide drug is preferably metformin, buformin, or a pharmaceutically acceptable salt thereof. This is because these drugs give satisfactory results as an oral hypoglycemic agent.

### BRIEF DESCRIPTION OF DRAWING

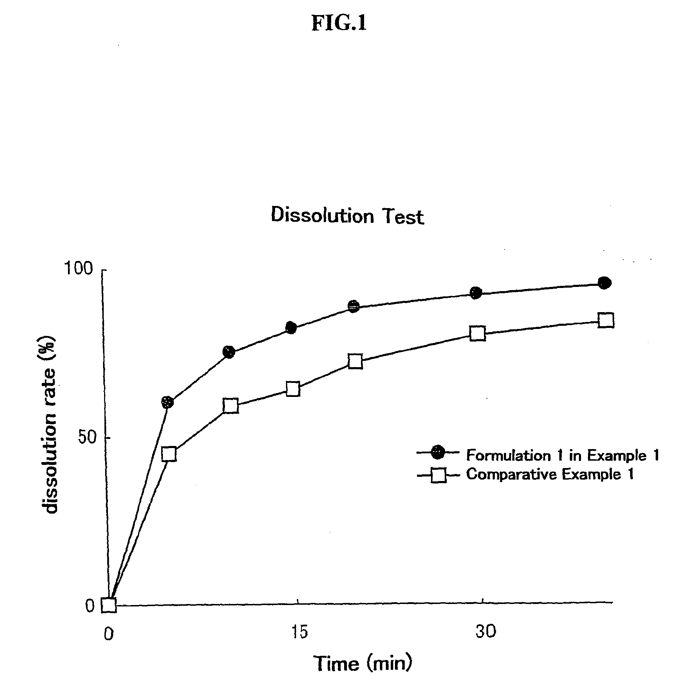

Fig. 1 shows the results of a dissolution test of metformin hydrochloride. A dissolution curve of a metformin hydrochloride-containing jelly preparation prepared using formulation 1 in Example 1 is shown in -●-, and a dissolution curve of a metformin hydrochloride-containing jelly preparation prepared in Comparative Example 1 is shown in -□-. The degree of dissolution (%) is shown on the ordinate, and the time (minutes) is shown on the abscissa.

### BEST MODE FOR CARRYING OUT THE INVENTION

Hereinafter, the embodiments and effect of the present invention are described.

The "biguanide drug" incorporated as a main agent into the jelly preparation of the present invention has an effect of not only suppressing gluconeogenesis to stimulate glycolysis action but also suppressing the absorption of glucose through the intestinal tract. The biguanide drug is used as a therapeutic agent for diabetes mellitus, but gives peculiar discomfort upon administration, and thus the suppression of such discomfort is needed. Such "biguanide drug" includes, for example, the drugs mentioned above.

The amount of the "biguanide drug" incorporated into one preparation varies depending on the kind of the biguanide drug or the dose per administration, and is suitably for example 200 to 2250 mg and can be suitably regulated.

The "inorganic acid" used in the present invention refers to a pharmaceutically acceptable inorganic acid capable of achieving an action for masking the harsh and bitter taste of the biguanide drug. The "inorganic acid", when added to the jelly preparation, can confer suitable disintegrating properties while maintaining the stability of the preparation, and thus rapid release of the drug in the digestive tract can be expected. Such "inorganic acid" includes, for example, the above-mentioned inorganic acids. The inorganic acid is preferably phosphoric acid and/or hydrochloric acid, and more preferably phosphoric acid.

The amount of the "inorganic acid" incorporated can be determined in such a range as to suppress the discomfort of the biguanide drug. For example, the ratio of the weight of the inorganic acid to the weight of the biguanide drug is preferably in the range of 0. 01 to 2. The lower limit is more preferably 0.03, 0.05, 0.1 or 0.15, and the upper limit is more preferably 1.5, 1.0 or 0.5. The "weight of the inorganic acid" used herein refers to the weight of substantially the acid only. When the inorganic acid is for example phosphoric acid that can be added as it is, the weight of the inorganic acid refers to the weight of the acid. However, when the inorganic acid is an acid such as hydrochloric acid that has been diluted with water or the like, the weight of the inorganic acid refers to the weight of the inorganic acid itself (hydrogen chloride in the case of hydrochloric acid) calculated from its normality or the like. When the biguanide drug added is a salt such as "metformin hydrochloride", the weight of the inorganic acid contained in the biguanide drug shall not be included in the "weight of the inorganic acid".

Further, a salt of the "inorganic acid" may be added. By adding the salt, a buffering action can be expected. Such salt includes, for example, a phosphate added in addition to phosphoric acid as the inorganic acid. The phosphate can improve the stability of the jelly preparation of the present invention by increasing the preparation pH decreased by adding phosphoric acid. Accordingly, the phosphate should show basicity upon dissolution in water, and is thus preferably a salt of phosphoric acid with a strong base. Such phosphate includes, for example, sodium hydrogenphosphate (Na₂HPO₄), sodium phosphate (Na₃PO₄), potassium hydrogenphosphate (K₂HPO₄), potassium phosphate (K₃PO₄), and a combination of two or more thereof.

An organic acid in the jelly preparation, on the other hand, could deteriorate the stability of the jelly preparation by inhibiting gelation, and it is thus not preferable to add an organic acid in a larger amount than necessary for exhibiting its characteristics (for example ascorbic acid as an antioxidant), and it is more preferable that an organic acid is substantially not added.

The "water-soluble polymer" for forming the preparation of the invention into a jelly preparation can be used without limitation insofar as it is a pharmaceutically acceptable water-soluble polymer capable of forming the jelly preparation mainly via gelation. Such "water-soluble polymer" includes, for example, polymers whose aqueous solutions gel by heating and subsequent cooling, such as carrageenan, agar, a combination of agar and carob bean gum, a combination of xanthane gum and carob bean gum, HM pectin, and LM pectin; polymers gelling by adding a divalent metal, such as LM pectin and sodium alginate; and polymers whose solutions are solidified by drying, such as pullulan. By using a mixture of two or more thereof, the gelling step can be facilitated and the ability for releasing a drug can be improved.

Although the amount of the "water-soluble polymer" added to one preparation varies depending on the kind of the water-soluble polymer, the desired degree of gelation, ability for releasing a drug, or the like, it is preferably 0.15 to 5.0 wt% based on the whole of the preparation. The lower limit is more preferably 0.2, 0.5, 0.7 or 1.0 wt%, and the upper limit is more preferably 4.0, 3.0 or 2.0 wt%.

The jelly preparation of the present invention may be blended with pharmaceutically acceptable additives. Such additives include, for example, sweeteners such as aspartame™, saccharine, sodium saccharine, stevia, erythritol, sorbitose, xylitol, reduced maltose syrup, or the like (preferably aspartame™, saccharine, sodium saccharine and/or stevia) ; flavors such as vanilla essence, lemon flavor, or the like; coloring agents; preservatives such as propyl p-oxybenzoate; and glycerin, and from these additives including the other additives, suitable additives may be selected and added.

For production of the jelly preparation of the present invention, a general method for producing jelly preparations can be used. For example, constituent components such as a biguanide drug and a water-soluble polymer are introduced into an aqueous solvent (limited to a pharmaceutically acceptable solvent) such aspurified water and distilled water in a stirring bath to form a substantially homogenous solution or a substantially homogenous dispersion at a predetermined temperature. The solution or dispersion is transferred to a high-temperature bath and sterilized by heating for example at 85°C for 30 minutes or more. Thereafter, the solution or dispersion is subjected to gelation depending on the water-soluble polymer added and then charged and packaged by a charging/packaging machine.

For production of the jelly preparation of the present invention, the pH of the preparation in the form of sol just before gelation is adjusted preferably to 4.0 or more. This is because when the pH is lower than 4.0, the jelly preparation is liable to cause syneresis, and stability of the preparation deteriorates in some cases. When the pH is 5.0 or more, the jelly preparation is more excellent in stability, and when the pH is 6.0 or more close to neutrality, the jelly preparation is far more excellent in stability. However, the pH is preferably 7.4 or less. This is because when the pH is over than 7.4, the jelly preparation may be accompanied by an amine smell in some cases.

Hereinafter, although the present invention is described in more detail by reference to Examples and Test Examples, the scope of the present invention is not limited thereto.

### Examples

### Example 1:

### Production of Metformin Hydrochloride-Containing Jelly

### Preparations

Metformin hydrochloride-containing jelly preparations were produced according to each of the formulations (wt%) in Table 1.

In formulation 1, for example, a metformin hydrochloride, a sodium hydrogen phosphate, a phosphoric acid and a sweetener were added to water, and stirred. Then, a carrageenan and a pullulan were added thereto, and stirred. Further, other ingredients were added thereto under stirring, and the mixture was heated to 85°C to form a homogenous solution. The solution was packaged in an aluminum laminate packaging container, and then left at room temperature to prepare a jelly preparation.

**Table 1**

| Ingredients | Formulation 1 | Formulation 2 | Formulation 3 | Formulation 4 | Formulation 5 | Formulation 6 |
|---|---|---|---|---|---|---|
| Metformin hydrochloride | 4.167 | 4.167 | 8.33 | 6.25 | 4.167 | 4.2 |
| Phosphoric acid | 0.833 | 1.25 | 0.4 | 0.85 | 0.4 | 0.8 |
| Sodium hydrogen phosphate | 1.667 | 2.5 | 1.2 | 2.0 | 1.0 | 4.4 |
| Xanthane gum | 0.08 | 0.3 | 0.5 | 0.4 | | 0.3 |
| Agar | 0.167 | | 0.5 | | | 0.2 |
| Carob bean gum | 0.083 | 0.15 | 0.5 | 0.2 | | 0.1 |
| Carrageenan | 0.833 | 0.1 | | | | 0.8 |
| Pullulan | 1 | | | | | |
| Gellan gum | | | | | 0.4 | |
| Sodium alginate | | 0.01 | | 0.2 | | |
| Pectin | | | 0.2 | | | |
| Sweetener | 0.083 | 0.1 | 0.05 | 0.01 | 0.2 | Suitable amount |
| Glycerin | 25 | 20 | 25 | 5 | 10 | 25 |
| Flavor | 0.01 | 0.01 | 0.01 | 0.01 | 0.01 | Suitable amount |
| Propyl p-oxybenzoate | 0.017 | 0.017 | 0.017 | 0.017 | 0.017 | Suitable amount |
| Water | Balance | Balance | Balance | Balance | Balance | Balance |
| Total | 100% | 100% | 100% | 100% | 100% | 100% |

### Example 2:

### Production of Buformin Hydrochloride-Containing Jelly

### Preparations

Buformin hydrochloride-containing jelly preparations were produced according to each of the formulations in Table 2.

**Table 2**

| Ingredients | Formulation 7 | Formulation 8 | Formulation 9 | Formulation 10 | Formulation 11 |
|---|---|---|---|---|---|
| Buformin hydrochloride | 10 | 10 | 12 | 15 | 8 |
| Phosphoric acid | 0.8 | 1.25 | 0.4 | 0.85 | 0.4 |
| Sodium hydrogen phosphate | 1.6 | 2.5 | 0.8 | 2.55 | 1.0 |
| Xanthane gum | 0.1 | 0.4 | 0.5 | 0.6 | |
| Agar | 0.15 | | 0.5 | | |
| Carob bean gum | 0.08 | 0.2 | 0.5 | 0.3 | 0.2 |
| Carrageenan | 0.83 | 0.05 | | | 0.6 |
| Sweetener | 0.08 | 0.1 | 0.05 | 0.01 | 0.2 |
| Glycerin | 25 | 20 | 25 | 5 | 10 |
| Flavor | 0.01 | 0.01 | 0.01 | 0.01 | 0.01 |
| Propyl p-oxybenzoate | 0.017 | 0.017 | 0.017 | 0.017 | 0.017 |
| Water | Balance | Balance | Balance | Balance | Balance |
| Total | 100% | 100% | 100% | 100% | 100% |

### Comparative Example 1

As a comparative example, a jelly preparation was produced using a formulation similar to the above formulation 1 except that an organic acid, that is a citric acid (0.8 wt%), was added in place of the inorganic acid, that is a phosphoric acid.

### Test Example 1:

### Dissolution test with the Metformin Hydrochloride-Containing Jelly Preparation

The metformin hydrochloride-containing jelly preparation prepared according to the formulation 1 in Example 1 and the metformin hydrochloride-containing jelly preparation prepared in Comparative Example 1 were examined for their ability for releasing a drug according to the dissolution test method 2, that is puddle method, described in B-679 and thereafter of "Japanese Pharmacopoeia, Fourteenth Edition".

Specifically, the solution 1 of the disintegration test under the Japanese Pharmacopoeia, which was assumed to be a gastric juice, was taken into a predetermined container, and adjusted to about 37°C. Next, each preparation was taken into the container, and then the dissolution rate (%) of the drug was determined at predetermined intervals. The results are shown in Fig. 1.

As shown in Fig. 1, while the metformin hydrochloride-containing jelly preparation having an organic acid was inferior in ability for releasing a drug, the ability for releasing a drug of the metformin hydrochloride-containing jelly preparation of the present invention was evidently improved.

### Test Example 2: Sensory Test

The metformin hydrochloride-containing jelly preparation with the formulation 1 and a commercial metformin-containing tablet were subjected to a sensory test by a panel of 10 persons. The amount of water taken at the time of administering the tablet was 10 mL which was the same amount of water contained in the jelly preparation. On the basis of both the physical aspect of jelly texture and tastes such as harsh or bitter taste, the sense upon administration was scored with the following three grades: "easy to swallow", "a little hard to swallow", and "hard to swallow". The results are shown in Table 3.

**Table 3**

| Sample | easily to swallow | a little hard to swallow | hard to swallow |
|---|---|---|---|
| Formulation 1 | 10 | 0 | 0 |
| Commercial Tablet | 0 | 3 | 7 |

As can be seen from the above results, the jelly preparation of the present invention is excellent in feeling upon eating, is also free of the harsh or bitter taste peculiar to the biguanide drug upon chewing, and can be easily swallowed.

### Test Example 3

The jelly preparation with the formulation 6 was examined in a stability test and a sensory test after the pH of the preparation before gelation was adjusted with phosphoric acid and sodium phosphate. The pH of the preparation with formulation 6 itself just before gelation was about 6.5.

The "stability test" was carried out in an accelerated condition in which the sample was left for 1 week under an atmosphere with 75% humidity and a temperature of 40°C, to examine the relationship between pH and syneresis.

The results of "sensory test" were evaluated in three grades, "excellent", "average" and "bad" with respect to feeling upon administration and tastes upon chewing. The results are shown in Table 4.

**Table 4**

| pH | Syneresis | Sensory test |
|---|---|---|
| 3.0 | occured | bad |
| 3.5 | occured | excellent |
| 4.0 | not occured | excellent |
| 4.5 | not occured | excellent |
| 5.0 | not occured | excellent |
| 5.5 | not occured | excellent |
| 5.8 | not occured | excellent |
| 6.0 | not occured | excellent |
| 6.5 | not occured | excellent |
| 7.4 | not occured | excellent |
| 7.6 | not occured | average |

The results shown above reveal that when the pH of the preparation with formulation 6 is less than 4.0, the stability of the gel is deteriorated. Accordingly, the pH is preferably 4.0 or more in consideration of stability. It was also revealed that when phosphoric acid is used as an agent for masking the bitter taste of the biguanide drug, the masking effect on the bitter taste is maintained even when the pH is 6 or more. On one hand, when the pH is 7.6, the preparation was accompanied by a slight amine smell.

It was thus revealed that the pH of the preparation containing metformin hydrochloride is preferably 4.0 or more, and more preferably 4.5 or more, to satisfy the contradictory characteristics of the preparation, namely stability and excellent administrability. In addition, the pH is preferably 6.0 or more, and 7. 4 or less for still higher stability of the preparation and for decreasing discomfort upon administration.

### INDUSTRIAL APPLICABILITY

Not only dose the biguanide drug-containing jelly preparation of the present invention make the discomfort upon administration decreased significantly, the preparation is excellent in stability and in ability for releasing a drug as well.

Accordingly, the biguanide drug-containing jelly preparation of the present invention is extremely excellent as a therapeutic agent for diabetes mellitus, which can easily secure patient's compliance.

## Claims

1. A biguanide drug-containing jelly preparation, comprising a biguanide drug, an inorganic acid, and a water-soluble polymer.

2. The biguanide drug-containing jelly preparation according to claim 1, wherein the weight ratio of the inorganic acid to the biguanide drug is in the range of 0.01 to 2.

3. The biguanide drug-containing jelly preparation according to claim 1 or 2, wherein pH of the preparation in a form of a solution just before gelation is 4.0 or more.

4. The biguanide drug-containing jelly preparation according to any one of claims 1 to 3, wherein the inorganic acid is phosphoric acid, hydrochloric acid, sulfuric acid, lower alkyl sulfonic acid, or a mixture of two or more selected therefrom.

5. The biguanide drug-containing jelly preparation according to any one of claims 1 to 3, wherein the inorganic acid is phosphoric acid.

6. The biguanide drug-containing jelly preparation according to claim 5, further comprising a phosphate.

7. The biguanide drug-containing jelly preparation according to any one of claims 1 to 6, wherein the water-soluble polymer is alginic acid, pectin, gellan gum, agar, carrageenan, carob bean gum, tamarind gum, pullulan, or a mixture of two or more selected therefrom.

8. The biguanide drug-containing jelly preparation according to any one of claims 1 to 7, wherein the biguanide drug is metformin, buformin, or a pharmaceutically acceptable salt thereof.
